# EUROPEAN PATENT APPLICATION

(11) **EP 4 160 211 A1**
(43) Date of publication of application: **05.04.2023**
(21) Application number: 21382877.5
(22) Date of filing: 29.09.2021
(51) Int. Cl.: G01N 33/564, C12N 5/071, G01N 33/76

(54) **DETECTING THE EXPOSURE OF A SUBJECT TO A THYROID DISRUPTING CHEMICAL, EVEN BEFORE HYPOTHYROIDISM APPEARS**

(71) Applicant: Moreno Navarro, José Carlos, 28029 Madrid (ES)
(72) Inventor: Moreno Navarro, José Carlos, 28029 Madrid (ES)
(74) Representative: Herrero & Asociados, S.L.

(57) **Abstract**

The invention relates to a method and biomarker for detecting the exposure of a subject to a Thyroid Disrupting Chemical, even before hypothyroidism appears, and the use thereof.

## Description

### FIELD THE INVENTION

The present invention belongs to the field of hypothyroidism. In particular, it refers to methods, markers and kits for detecting the exposure of a subject to a Thyroid Disrupting Chemical and their use for hypothyroidism diagnosis and characterisation.

### BACKGROUND

Endocrine-disruptor chemicals (EDC) are a cause of concern for Public Health. They are basically anthropogenic products derived from industrial activities like plastics, pesticides, insecticides, flame retardants or food colorants polluting the human milieu. In particular, halogenated (iodinated, brominated, chlorinated or fluorinated) aromatic compounds are abundant EDCs that can alter the normal physiology of hormone systems. When they alter the thyroid hormone axis, they are named thyroid disrupting chemicals (TDCs) (1,2). Thyroid hormone is essential for the correct development of the fetal brain, and failure of T4 to cross the placenta in enough quantities to reach the fetal circulation and the developing brain is associated with neurodevelopmental disorders like attention deficit and hyperactivity disorders (ADHD) and autistic spectrum disorders (ASD) (1,2). Halogenated TDC are extensively present in our daily lives as components of widely used consumer products. Many of them alter the blood levels of TSH, T4 or T3, causing mainly hypothyroidism. In animal research, some mechanisms have been described, like binding of TDCs to serum proteins transporting T4 in the blood stream, inhibition of enzymes involved in activation of T4 to T3 by deiodination, or interaction with the thyroid hormone receptors (1,2).

Deep concerns have raised in Public Health regarding the pathogenic effects of TDCs in the general population, so legal frameworks have been established by governments in order to diminish the adverse effects of these worldwide used agents (3). However, such measures are difficult to implement in an individual basis due to the lack of knowledge on compounds amenable to determination that can safely indicate exposure to chemical(s) is occurring, the so called named "biomarkers". To our knowledge, there is no available biomarker that can flag the exposure of a given individual to a TDC source at the time that endocrine damage (e.g. hypothyroidism) is diagnosed and could thus be used to suspect that the etiology of hypothyroidism could be environmental (versus other, e.g. genetic, causes) explaining the disorder in clinical practice. The use of such biomarkers in clinical practice would allow investigation and implementation of prevention measures against damaging TDCs sources. The lack of biomarkers to flag environmental damage prevents establishing the environmental etiology and investigating and detecting harming pollutants in routine practice. A major drawback is the fact that there are myriads of chemicals that humans may be currently exposed to, sometimes in combination, and current methods for direct TDC detection (basically Gas or Liquid Chromatography with tandem Mass Spectrometry, GS/MS-MS or LC/MS-MS, respectively) are mainly useful in studies on populations living in geographical areas with suspected contamination by a (a priori) pre-established set of compounds (4,5,6). In parallel to chemical detection, mean hormone levels in the studied population are determined and statistical correlations are calculated (4,5,6). This approach is typically epidemiological but lacks diagnostic individual value. Furthermore, the methods are expensive and difficult to implement in analytical routines in hospitals. Therefore, the current technical development halts the practical possibility of an environmental diagnose in the outpatient clinic and, as consequence, the adoption of individual measures to a) identify particular compounds causing the disorder and b) reduce the exposure of a person to its particular contaminating source. Thus, the development of Environmental Medicine urgently demands the identification of biomarkers with individual diagnostic value that are applicable in the clinical setting.

### OBJECT OF THE INVENTION

The present invention refers in a **first aspect** to a method for detecting the exposure of a subject to a Thyroid Disrupting Chemical (TDC), comprising determining the concentration of mono-iodotyrosines (MIT) and di-iodotyrosines (DIT) in a biological sample taken from said subject, wherein an increase in the concentration of MIT and a decrease in the concentration of DIT in comparison to a reference is indicative of exposure to a TDC, or wherein an increased ratio MIT/DIT in comparison to a reference is indicative of exposure to a TDC..

A **second aspect** of the invention refers to a method for determining the cause of non-genetic hypothyroidism in a subject comprising determining the concentration of MIT and DIT in a biological sample taken from said subject, wherein an increase in the concentration of MIT and a decrease in the concentration of DIT in comparison to a reference is indicative of said hypothyroidism being caused by exposure to a TDC, or wherein an increased ratio MIT/DIT in comparison to a reference is indicative of said hypothyroidism being caused by exposure to a TDC.

In a **third aspect,** the present invention refers to the use of a kit for carrying out the method of any one of the first and second aspects of the invention, wherein the kit comprises:
a) appropriate means for determining the concentration of MIT,
b) appropriate means for determining the concentration of DIT.

In a **fourth aspect,** the present invention refers to the use of a method according to any one of the embodiments of the first and/or second aspect of invention, or of a kit as defined in any one of the embodiments of the third aspect of the invention, for diagnosis of hypothyroidism caused by exposure to TDC (TDC-caused hypothyroidism), for detecting the exposure of a subject to a TDC, or for determination of the cause of non-genetic hypothyroidism.

In a **fifth aspect** the present invention relates to a method for preventing hypothyroidism caused by exposure to TDC comprising determining the concentration of MIT and DIT in a biological sample taken from said subject, wherein an increased in the concentration of MIT and a decreased in the concentration of DIT in comparison to a reference is indicative that the subject should avoid the source of the TDC.

Other objects, features, advantages and aspects of the present application will be apparent to the person skilled in the art from the following description and appended claims.

### BRIEF DESCRIPTION OF THE FIGURES

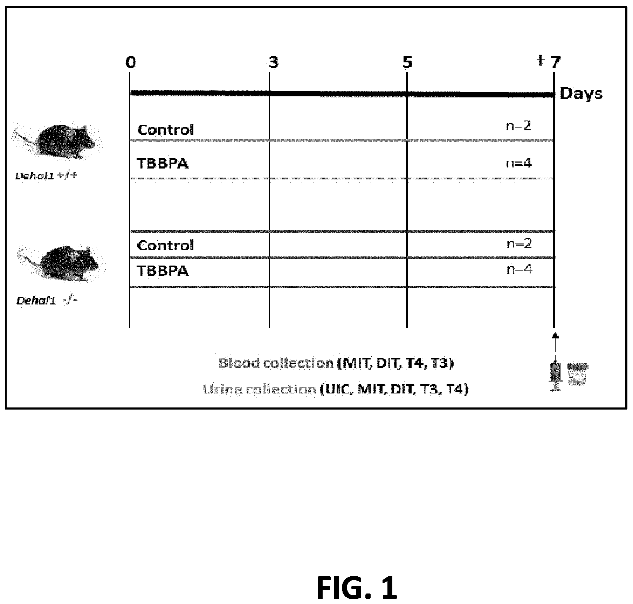

**Figure 1****:** *In vivo* experimental design. Two female animals per genotype-control groups and 4 animals per TBBPA groups were used. The controls were treated only with vehicle while the study cases were treated with the vehicle containing TBBPA or MNT.

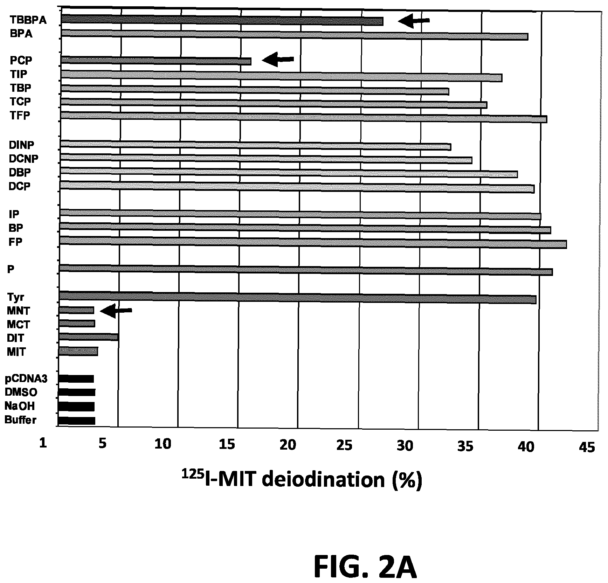

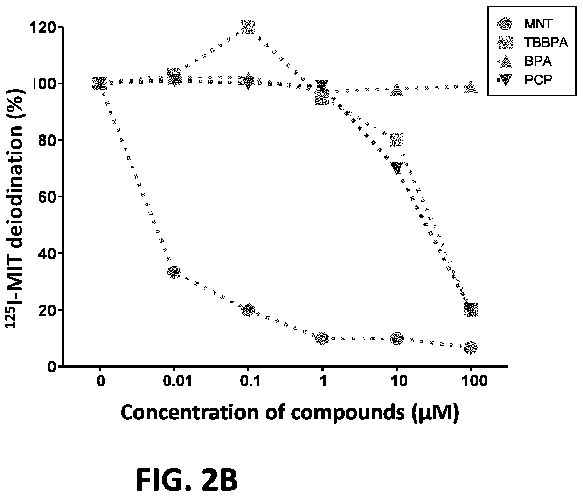

**Figure 2****:** Inhibition profiles of xenobiotic compounds on Dehal1 activity. (A) Screening of a battery of halogenated or nitrated phenolic compounds (at 10 µM concentration) for inhibition of ¹²⁵I-MIT deiodination by Dehal1. (B) Inhibition curve of 3 compounds shown capable to inhibit Dehal1 activity at the screening (MNT (red circle), PCP (blue down-looking triangle), TBBPA (orange square)) and BPA (bisphenol A, pink up-looking triangle) as controls for a non-halogenated phenolic compound.

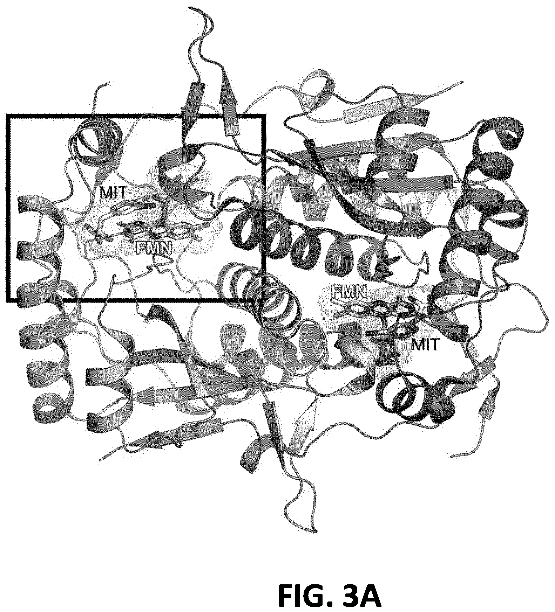

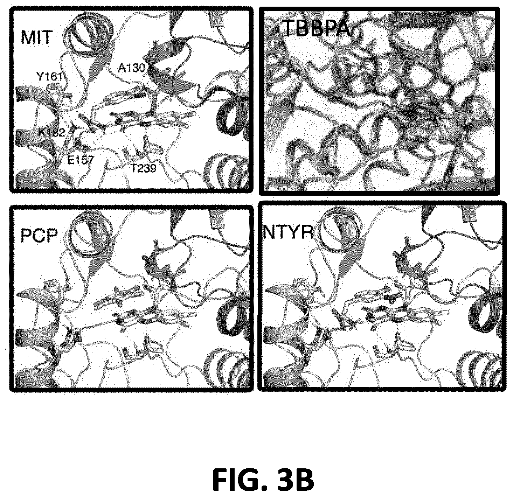

**Figure 3****:** Molecular docking of phenolic compounds with the dimeric crystallographic structure of DEHAL1. (A) MIT interacts with the 2 substrate binding pockets in the Dehal1 dimer. (B) Details of the interaction of MIT, TBBPA, PCP and MNT (NTYR) within the substrate binding pocket of the enzyme, supporting competitive inhibition of the three compounds for MIT (natural substrate) deiodination.

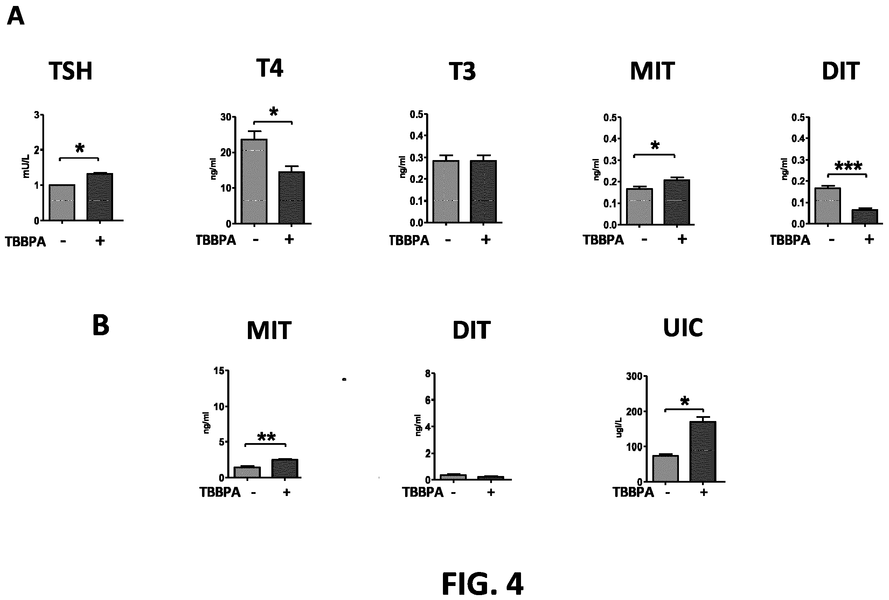

**Figure 4****:** Graphic representation of the concentration of TSH, T4, T3, MIT, DIT in mice exposed to TBBPA (+) or not exposed, in plasma (A) and urine (B).

### DESCRIPTION OF THE INVENTION

As used in the present application, the singular forms, e.g. "a", "one" and "the" include their corresponding plurals unless the context clearly indicates otherwise. Unless otherwise defined, all technical and scientific terms used in this document have the meaning commonly understood by one of ordinarily skilled in the art to which this invention belongs.

To facilitate understanding and clarify the meaning of specific terms in the context of the present invention, the following definitions and particular and preferred embodiments thereof, applicable to all the embodiments of the different aspects of the present invention, are provided:
Endocrine-disrupting chemicals (EDCs) are chemicals that mimic, block, or interfere with hormones in the body's endocrine system. The definition provided by the American Endocrine Society applies to the present invention. Said definition is: "An endocrine-disrupting chemical (EDC) is an exogenous chemical, or mixture of chemicals, that can interfere with any aspect of hormone action, including regulation of hormone synthesis, secretion, and effects and the variability in regulation of these events across the life cycle". Likewise, TDCs are exogenous chemicals that mimic, block or interfere with the thyroid hormone axis of the body.

As used herein, the term "subject" refers to a member of the class Mammalia, including, without limitation, human and non-human primates such as chimpazees and other apes and monkey species; farm animal such as cattle, sheep, pigs, goats and horses; domestic mammals such as dogs and cats; laboratory animals including rodents such as mice, rats and guinea pigs, and the like. The subject is preferably a human.

A "biological sample" in terms of the present invention means a sample of biological fluid. In a preferred embodiment according to any of the aspects of the invention, the biological fluid such as sputum, saliva, whole blood, serum, plasma, urine, cerebrospinal fluid, preferably whole blood, plasma or urine which, advantageously, are non- or minimally invasive.

In the context of the present invention the term "reference" refers to a value or level, which has been determined by measuring the concentration of MIT and DIT (or any other marker) as the test sample in a biological sample taken from a subject or a population of subjects not suffering from hypothyroidism, i.e. hypothyroidism-free subject/population. The sample taken from a hypothyroidism-free subject is also referred as "control sample", thus, reference also refers to concentration level of a control sample. Preferably, the control sample is a sample of subjects matched on age and body mass index, and from the same location, to the subject analysed. Preferably, the reference is a reference value, a cut-off value or a threshold.

As shown in the examples below, it was found that changes in MIT and DIT concentration can be efficiently used as a predictive biomarker for the exposure to TDCs.

Thus, in a **first aspect**, the present invention refers to a method, particularly an *in vitro* method, for detecting the exposure of a subject to a TDC, essentially consisting of, or consisting of, or comprising determining the concentration of mono-iodotyrosines (MIT) and di-iodotyrosines (DIT) in a biological sample taken from said subject, wherein an increase in the concentration of MIT and a decrease in the concentration of DIT in comparison to a reference is indicative of exposure to a TDC, or wherein an increased ratio MIT/DIT in comparison to a reference is indicative of exposure to a TDC.

In a **second aspect,** the present invention relates to a method, in particular an *in vitro* method, for determining the cause of non-genetic hypothyroidism in a subject essentially consisting of, or consisting of, or comprising determining the concentration of MIT and DIT in a biological sample taken from said subject, wherein an increase in the concentration of MIT and a decrease in the concentration of DIT in comparison to a reference is indicative of said hypothyroidism being caused by exposure to a TDC, or wherein an increased ratio MIT/DIT in comparison to a reference is indicative of said hypothyroidism being caused by exposure to a TDC.

As shown in the examples, variations in MIT and DIT can (bio)mark (and even anticipate) hypothyroidism caused by environmental exposure to TDCs in individual determination of their concentrations or ratio, and comparison to mean levels in the geographical area they live in.

In a particular embodiment of the method of the first and second aspects of the invention, the TDC inhibits Dehal1.

In another particular embodiment of the method of the first and second aspects of the invention according any of to the above-mentioned embodiments, the TDC is a phenolic compound with (2) halogen- or nitro- substitution, and (2) a hydroxyl group substitution.

In another particular embodiment of the method of the first and second aspects of the invention according to the preceding embodiments, the TDC is selected from the group consisting of tetrabromobisphenol A (TBBPA); pentachloro-phenol (PCP); 3-L-nitrotyrosine (MNT); polychlorinated biphenyls; polybrominated diphenyl ethers; Bromoxynil; Oxyclozanide; Bithionol; Tribromsalan; Nitroxynil; Closantel; Benzbromarone; Triclosan; Rose Bengal; Erythrosine B; Phloxine B; Methylene blue; Tannic acid; Diquat dibromide monohydrate; D&C Red 27; Dodecylphenol; Lauryl gallate; 4-Dodecylphenol; 4-Nonylphenol; Dinocap; Hexadecyltrimethyl-ammonium bromide Sodium myristyl sulfate; Calcium dodecylbenzene sulfonate; 2,2'-Methylenebis(4-methyl-6-tert- butylphenol); Sodium hexyldecyl sulfate; Sodium tridecyl sulfate; Sodium dodecylbenzenesulfonate; Methylbenzethonium chloride; Docusate sodium; Kepone; Oleic acid; Dodecylbenzenesulfonic acid; Octylparaben; C.I. Acid Red 114; and combinations thereof.

More particularly, the TDC is selected from the group consisting of TBBPA; PCP; MNT; 4-OH-2',3,4',5,6'-pentachlorobiphenyl; 4-OH-2',3,4',6-tetrachlorobiphenyl; 4-OH-2,2,5,5-tetrachlorobiphenyl; 4,4'-diOH-3,3',5,5'-tetrachlorobiphenyl; 3-OH-2,2',5,5'-tetrachlorobiphenyl; 4'-OH-brominated diphenyl ether (BDE)-17; 4-OH-BDE-42; 4'-OH-BDE-49; 4-OH-BDE-90; 2-OH-BDE-15; 2'-OH-BDE-28; Bromoxynil; Oxyclozanide; Bithionol; Tribromsalan; Nitroxynil; Closantel; Benzbromarone; Triclosan; Rose Bengal; Erythrosine B; Phloxine B, and combinations thereof. Preferably, the TDC is TBBPA, PCP, MNT or a combination thereof, more preferably TBBPA and/or PCP.

TBBPA and PCP are recognized TDCs since they were shown in the past to alter thyroid hormone levels. MNT is a signaling molecule in the human organism (endogenous) that is related to situations of oxidative stress in the cell. However, the recent identification of MNT in polluted air and atmospheres (from anthropogenic activities, derived from combustion processes in thermal power station or from cars) make it also an exogenous chemical, strongly suggesting that MNT can also be considered a polluting chemical in the atmosphere that humans are exposed to, fitting with the definition of TDC provided its well-known properties as inhibitor of Dehal1 *in vitro* and *in vivo,* together with the present data showing that, even at low dose exposure, mice have experimented disturbed iodotyrosine levels, especially MIT, when they are exposed to MNT. Based on above data, these authors support the categorization of MNT as an TDC.

In a particular embodiment of the method of the first and second aspects of the invention according to any one of the embodiments mentioned above, the concentration of MIT of the test sample is higher than (increased over) the concentration of the control sample, when it is at least 10%, at least 20%, at least 25%, at least 30%, at least 35%, at least 40%, at least 45%, at least 50%, at least 55%, at least 60%, at least 65%, at least 70%, at least 75%, at least 80%, at least 85%, at least 90%, at least 95%, at least 100% higher than in the control sample. Preferably it is at least 20% higher.

In a particular embodiment of the method of the first and second aspects of the invention according to any one of the embodiments mentioned above, the concentration of MIT in the test sample is considered to be higher than (increased over) the reference when the differences in average beta-values between groups (hypothyroidism and non-hypothyroidism) is higher than a set threshold, preferably higher than 0.20.

In a particular embodiment of the method of the first and second aspects of the invention according to any one of the embodiments mentioned above, the concentration of DIT of the test sample is lower than (decreased over) the concentration of the control sample, when it is at least 10%, at least 15%, at least 20%, at least 25%, at least 30%, at least 35%, at least 40%, at least 45%, at least 50%, at least 55%, at least 60%, at least 65%, at least 70%, at least 75%, at least 80%, at least 85%, at least 90%, at least 95%, at least 100% lower than in the control sample. Preferably it is at least 15% lower.

In a particular embodiment of the method of the first and second aspects of the invention according to any one of the embodiments mentioned above, the concentration of MIT in the test sample is considered to be higher than (decreased over) the reference when the differences in average beta-values between groups (hypothyroidism and non-hypothyroidism) is lower than a set threshold, preferably higher than 0.20.

In a particular embodiment of the method of the first and second aspects of the invention according to any one of the embodiments mentioned above, the ratio MIT/DIT of the test sample is higher than (increased over) the ratio of the control sample, when it is at least 40%, at least 50%, at least 60%, at least 70%, at least 80%, at least 90%, at least 100%, at least 120%, at least 150%, at least 170%, at least 200%, at least 220%, at least 240%, higher than in the control sample. Preferably it is at least 70% higher.

In a particular embodiment of the method of the first and second aspects of the invention according to any one of the embodiments mentioned above, the sample is urine, the TDC is MNT and the ratio is increased at least 80% over the control sample.

In a particular embodiment of the method of the first and second aspects of the invention according to any one of the embodiments mentioned above, the sample is urine, the TDC is TBBPA and the ratio is increased at least 150%, preferably at least 170%, over the control sample.

In a particular embodiment of the method of the first and second aspects of the invention according to any one of the embodiments mentioned above, the sample is plasma, the TDC is TBBPA and the ratio is increased at least 200%, preferably at least 240%, over the control sample.

In a particular embodiment of the method of the first and second aspects of the invention according to any one of the embodiments mentioned above, the method further consists essentially of, or consists of, or comprises determining the median urinary iodine concentration (UIC).

In a particular embodiment of the method of the first and second aspects of the invention according to any one of the embodiments mentioned above, the method further consists essentially of, or consists of, or comprises determining the concentration of thyroxine (T4) and triiodothyronine (T3).

In a particular embodiment of the method of the first and second aspects of the invention according to any one of the embodiments mentioned above, the concentration of MIT and DIT, and optionally T4 and/or T3, is determined simultaneously, preferably by high performance liquid chromatography coupled to tandem mass spectroscopy (HPLC/MS-MS). More particularly, it is determined as described in the Examples.

In a particular embodiment of the method of the first and second aspects of the invention according to any one of the embodiments mentioned above, the method further consists essentially of, or consists of, or comprises the determination of thyroid-stimulating hormone (TSH).

The methods of the invention can be carried out using a kit. Thus, in a third aspect, the present invention refers to the use of a kit for carrying out the method of any one of the embodiments of the first and/or second aspect of the invention, wherein the kit comprises:
a) appropriate means for determining the concentration of MIT, and
b) appropriate means for determining the concentration of DIT.

In a particular embodiment, the kit further comprises appropriate means for determining the concentration of T4 and T3.

In another particular embodiment according to any one of the preceding embodiments, the kit further comprises appropriate means for determining UIC and/or TSH.

In another particular embodiment according to any one of the preceding embodiments, the kit further comprises a suitable packaging. Additionally, the kit can contain instructions for the simultaneous, sequential or separate use of the different components which are in the kit.

In a **fourth aspect** the present invention refers to the use of a method according to any one of the embodiments of the first and/or second aspect of invention, or of a kit as defined in any one of the embodiments of the third aspect of the invention, for diagnosis, particularly *in vitro* diagnosis, of TDC-caused hypothyroidism, or for detecting the exposure of a subject to a TDC, or for determination of the causes of non-genetic hypothyroidism. Preferably the use of the fourth aspect of the invention is an *in vitro* use.

The fourth aspect of the invention refers also to MIT and DIT as (bio)markers for TDC-caused hypothyroidism *in vitro* diagnosis, or for *in vitro* detecting the exposure of a subject to a TDC, or for *in vitro* determination of the causes of non-genetic hypothyroidism. The fourth aspect also refers to MIT and DIT for use in TDC-caused hypothyroidism diagnosis, or for use in detecting the exposure of a subject to a TDC, or for use in the determination of the causes of non-genetic hypothyroidism.

In a **fifth aspect** the present invention relates to a method for preventing hypothyroidism caused by exposure to TDC comprising determining the concentration of MIT and DIT in a biological sample taken from said subject, wherein an increased in the concentration of MIT and a decreased in the concentration of DIT in comparison to a reference or an increased ratio MIT/DIT is indicative that the subject should avoid the source of the TDC.

The fifth aspect of the invention refers also to MIT and DIT as (bio)markers for prevention of TDC-caused hypothyroidism, and to MIT and DIT for use, as (bio)markers, for prevention of non-genetic TDC-caused hypothyroidism. An increase in the concentration of MIT and a decrease in the concentration of DIT in comparison to a reference or an increased ratio MIT/DIT is indicative that the subject should avoid the source of the TDC in order to prevent hypothyroidism caused by exposure to TDC.

The particular and preferred embodiments described in the first and second aspects of the invention are applicable to the third, fourth and fifth aspects of the invention.

All publications mentioned herein are hereby incorporated in their entirety by reference. While the foregoing invention has been described in some detail for purposes of clarity and understanding, it will be appreciated by one skilled in the art from a reading of this disclosure that various changes in form and detail can be made without departing from the true scope of the invention and appended claims.

The examples below serve to further illustrate the invention, and to provide those of ordinary skill in the art with a complete disclosure and description of how the methods and uses herein are carried out and are not intended to limit the scope of the present invention.

### REFERENCES:

1.Mughal BB, Fini JB, Demeneix BA. Thyroid-disrupting chemicals and brain development: an update. Endocr Connect. 2018; 7(4): R160-R186.
2.Barbara A Demeneix. Evidence for Prenatal Exposure to Thyroid Disruptors and Adverse Effects on Brain Development. Eur Thyroid J. 2019; 8(6):283-292.
3.Leung AM, Korevaar TI, Peeters RP, Zoeller RT, Köhrle J, Duntas LH, Brent GA, Demeneix BA. Exposure to Thyroid-Disrupting Chemicals: A Transatlantic Call for Action. Thyroid. 2016; 26(4):479-80.
4.Lopez-Espinosa MJ et al. Association between thyroid hormone levels and 4,4'-DDE concentrations in pregnant women (Valencia,Spain). Environ Res. 2009; 109(4):479-85.
5.Alvarez-Pedrerol M et al. (2006). The association between organochlorine and thyroid hormone levels in cord serum: A study from northern Thailand. Environ Int.; 32:554 -559.
6.Chevrier J et al. (2008). Effects of exposure to polychlorinated biphenyls and organochlorine pesticides on thyroid function during pregnancy. Am J Epidemiol.; 168(3):298-310.
7.Moreno JC. Identification of novel genes involved in congenital hypothyroidism using Serial Analysis of Gene Expression. Horm. Res. 2003; 60: 96- 102.
8.Moreno JC, Klootwijk W, van Toor H, et al. Mutations in the iodotyrosine deiodinase gene and hypothyroidism. N Engl J Med. 2008; 24; 358: 1811-8.
9.Moreno JC, Visser TJ. Genetics and phenomics of hypothyroidism and goiter due to iodotyrosine deiodinase (DEHAL1) gene mutations. Mol Cell Endocrinol.;322 (1-2):91-8. 2010.
10.McTamney PM, Rokita SE. A mammalian reductive deiodinase has broad power to dehalogenate chlorinated and brominated substrates. J Am Chem Soc. 2009; 131(40):14212-3.
11.Mani AR, Moreno JC, Visser TJ, Moore KP. The metabolism and de-bromination of bromotyrosine in vivo. Free Radic Biol Med. 2016; 90, pp. 243 - 251.
12.Su Q, Boucher PA, Rokita SE. Conversion of a Dehalogenase into a Nitroreductase by Swapping its Flavin Cofactor with a 5-Deazaflavin Analogue. Angew Chem Int Ed Engl. 2017 28;56(36):10862-10866.
13.Shimizu R, Yamaguchi M, Uramaru N, Kuroki H, Ohta S, Kitamura S, Sugihara K. Structure-activity relationships of 44 halogenated compounds for iodotyrosine deiodinase-inhibitory activity. Toxicology. 2013;314(1): 22-9.
14.Olker JH, Korte JJ, Denny JS, Haselman JT, Hartig PC, Cardon MC, Hornung MW, Degitz SJ. In vitro screening for chemical inhibition of the iodide recycling enzyme, iodotyrosine deiodinase. Toxicol In Vitro. 2021; 71:105073.
15.lto T, Ogino K, Nagaoka K, Takemoto K, Nishiyama R, Shimizu Y. Detection of 3-Nitrotyrosine in Atmospheric Environments via a High-performance Liquid Chromatography-electrochemical Detector System. J Vis Exp. 2019; 30; (143).
16.Thomas SR, McTamney PM, Adler JM, Laronde-Leblanc N, Rokita SE. Crystal structure of iodotyrosine deiodinase, a novel flavoprotein responsible for iodide salvage in thyroid glands J Biol Chem. 2009; 284(29):19659-67.
17.Robichaux, J.P., Le, X., Vijayan, R.S.K. et al. Structure-based classification predicts drug response in EGFR-mutant NSCLC. Nature (2021). https://doi.org/10.1038/s41586-021-03898-1.
18.Saba A, Donzelli R, Colligiani D, Raffaelli A, Nannipieri M, Kusmic C, Dos Remedios CG, Simonides WS, lervasi G, Zucchi R. Quantification of thyroxine and 3,5,3'-triiodo-thyronine in human and animal hearts by a novel liquid chromatography-tandem mass spectrometry method. Horm Metab Res 2014;46(9):628-34.
19.Matuszewski BK, Constanzer ML, Chavez-Eng CM. Strategies for the assessment of matrix effect in quantitative bioanalytical methods based on HPLC-MS/MS. Anal Chem. 2003;75(13):3019-30.
20.Borsò M, Agretti P, Zucchi R, Saba A. Mass spectrometry in the diagnosis of thyroid disease and in the study of thyroid hormone metabolism Mass Spectrom Rev. 2020 Nov 25. doi: 10.1002/mas.21673.
21.Benotti J, Benotti N. Protien-bound iodine, total iodine, and butanol-extractable iodine by automation. Clin Chem 1963; 12:408-416.

### EXAMPLES

### Example 1. MATERIALS AND METHODS

### 1.1. Cell culture and transfection assays.

Human embryonic kidney (HEK293) cells were grown in Dulbecco's modified Eagle medium (Gibco) supplemented with 10% fetal calf serum and 0.1 mM flavin mononucleotide (FMN, MP Biochemicals) in 6-well plates. At 50% confluency, cells were transfected with 1 µg of wild-type or mutant DEHAL1 plasmid or empty pcDNA3.1 plasmid, using Fugene6 (Roche). After 24 h incubation, cells were washed with 1 ml PBS and collected in 0.5 ml PED buffer (0.1 M phosphate, pH 7.2, 2 mM EDTA, 1 mM dithiothreitol) per well and kept on ice. Samples were sonicated twice for 10 sec using Soniprep150, frozen on dry ice-ethanol and stored at -80 °C. For experiments testing FMN-response, culture medium was supplemented with 0-2 mM FMN.

### 1.2. In vitro Dehal1 activity assay.

The experiments follow described methods to determine the MIT deiodination capacity of Dehal1-transfected cells (8), adding the candidate chemicals to test their capacity to inhibit such reaction. Basically, labelled [¹²⁵I]MIT was prepared by radioiodination of L-tyrosine (Sigma-Aldrich) with ¹²⁵I - (specific activity: 15 Ci/mg, Amersham Biosciences) using the chloramine-T method and purified by chromatography on cation-exchange resin (Dowex 50WX2-200, Sigma-Aldrich). HPLC analysis on a C18 reversed-phase column showed 96% purity of [¹²⁵I]MIT, with trace amounts of ¹²⁵-I⁻. Incubations contained 105 cpm [¹²⁵I]MIT, 0.1 µM unlabeled MIT and 10-25 µg cell homogenate protein in a final volume of 0.1 ml PED buffer. Reactions were started by the addition of NADPH (final concentration 0.1 mM), and the mixtures were incubated for 60 min at 37°C. Reactions were stopped by the addition of 0.9 ml of 10% acetic acid on ice. Samples were applied to 2 ml Dowex 50WX2-200 columns, and successively eluted with 3x1 ml 10% acetic acid (containing eluted iodide), 1 ml water, and 4×1ml 1 M NH4OH (containing iodotyrosines). Radioactivity was measured in each fraction in a NE1600 gamma counter (NE Technologies) and percentage deiodination was calculated. Protein concentrations in cell homogenates were measured by Bradford's assay; iodotyrosine deiodinase activity was corrected for the slight 1251 - production in empty pcDNA3.1-transfected cell homogenates and expressed per mg protein. With [¹²⁵I]DIT as the substrate, calculation of deiodinase activity took into consideration that [¹²⁵I]MIT did not significantly accumulate during the reaction but was rapidly further deiodinated.

### 1.3. In vitro Dehal1 inhibition assays.

For the general screen of inhibition potential of each compound, 10 µM unlabeled chemicals tested (Sigma-Aldrich) were added to the incubation mixture together with 10 µM unlabeled MIT with no further modifications on the protocol above. Said chemical tested were: Tetrabromobisphenol A (TBBPA), Bisphenol A (BPA), Pentachlorophenol (PCP), tetraiodophenol (TIP), Tetrabromophenol (TBP), tetrachlorophenol (TCP), tetraafluorophenol (TFP), Di-iodo-nitro-Pheol (DINP), Di-chloro-nitrophenol (DCNP), Dibromophenol (DBP), Diclorophenol (DCP), iodophenol (IP), Bromophenol (BP), Fluorophenol (FP), phenol (P), Tyrosien(Tyr), mononitrotyrosine (MNT), monochlorotyrosine (MCT), Monoiodotyrosine (MIT), Diiodotyrosine (DIT). As negative controls: empty pcDNA-transfected cells, DMSO, NaOH and Buffer.

Crude deiodination capacity obtained in individual chemical-containing mixture was calculated, including the vehicle (DMSO)-containing mixture. Percentage inhibition of each compound was determined with respect to the vehicle containing mixture. For inhibition curves of particular compounds, equimolar concentrations of unlabeled MIT (0, 0.1, 1, 10, 100 µM) and BPA, TBBPA, PCP and MNT were used, and % inhibition of individual compounds was calculated as above.

### 1.4. In silico molecular docking studies

The Crystal structure of iodotyrosine deiodinase (IYD) [Protein Data Bank Identification (PDB ID): C217A] was used (16). MIT, DIT, TBBPA, PCP and MNT were docked into the "active-like" conformation of IYD using MOE (Chemical Computing Group Inc., Montreal, QC, Canada) (17). MIT, DIT and the putative inhibitor chemicals was docked into the "active" conformation of IYD using MOE (Chemical computing group Inc., Montreal, QC, Canada) in such a manner that all chemical interactions between MIT, FMN and IYD neighbouring amino acids remain, as described (16).

### 1.5. In vivo studies.

Mice. Female wild type mice from C57BI/6 J strain were purchased from Charles River company. Animals were maintained under standard conditions of temperature (22-24°C), humidity (40-50%), 12-12 hours of dark/light cycle, water and rodent diet *ad libitum.* Five months old female mice were acclimatized prior to the experiment and handled based on animal bioethical protocols. The experimental procedures were approved by the Research Ethical Committee of the Autonomous University of Madrid and the competent department of Comunidad de Madrid. Tetrabromobisphenol A powder (TBBPA, 97% purity) obtained through Sigma-Aldrich was dissolved in a vehicle containing 25% ethanol and 75% of peanut oil. A TBBPA dose of 500 mg/kg of body weight was injected subcutaneously for 7 days. Blood and urine collection were performed at the end of the experiment (Fig. 1).

**Blood, urine and tissue collection.** At day 7, blood was collected from cardiac puncture in heparinized tubes and centrifuged (2000 g for 10 minutes) to isolate plasma. Urine was collected using hydrophobic sand (Coastline Global, USA). Samples were kept at -20°C until used. For tissue isolation, mice were kept under deep anesthesia (Sevorane) and euthanized by cervical dislocation.

### Measurement of MIT, DIT, T3, and T4 in plasma and/or urine.

Previously published methods [18, 19] were modified to allow the simultaneous quantification of MIT, DIT, T3, T4.

*Sample preparation:* 100 µL of urine/plasma were added with suitable amounts of stable isotope-labeled internal standards (5.4 pmol 13C9-MIT, 4.0 pmol 13C9-DIT, 2.8 pmol 13C6-T3, 2.4 pmol 13C6-T4), vortexed, stored 30 min at room temperature to equilibrate, and deproteinized with the addition of 300µL of cold-acetone and the storage at 4°C for 30 min. After centrifugation at 22780 × g for 10 min, supernatants were dried at 40 °C under a gentle stream of nitrogen, derivatized with 200 µL of 3.0 N hydrochloric acid in n-butanol and incubated for 45 min at 60 °C, for the formation of the corresponding butyl esters of tyrosines, thyroid hormones, and their internal standards. After further evaporation to dryness, the sample was reconstituted with 500 µL of 0,1 M potassium buffer (pH=4) and loaded onto Agilent (Santa Clara, CA, USA) Bond-Elut Certify 130 mg SPE cartridges, to perform the extraction according to the procedure described by *Saba et al.* [20]. The eluate was dried once again under nitrogen, the dried residue was reconstituted with 100 µL of acetonitrile/0.1 M hydrochloric acid (50/50 by volume), and 1µL of it was injected into the HPLC-MS-MS system. Calibration curves ranged from 0.1 to 100.0 ng/mL and were daily prepared by serial dilution in methanol and derivatized with samples.

*Instrument layout:* AB-Sciex (Concord, Ontario, Canada) QTRAP 6500+ triple quadrupole mass spectrometer with ESI source, coupled to an Agilent (Santa Clara, CA, USA) 1290 UHPLC system, fitted with a 110 Å, 2x50 mm, 3µm particle size, Gemini C18 column (Phenomenex, Torrance, CA).

*Operative conditions:* the chromatographic separation was carried out at 400 µL/min as a flow rate, using methanol/acetonitrile (20/80, by volume) added with 0.1% formic acid (solvent A) and water containing 0.1% formic acid (solvent B). The elution gradient was the following: 95 % solvent B from 0 to 3 min; 35 % solvent B from 3 to 8.5 min; 0 % solvent B from 8.5 to 9 min; 0 % solvent B from 9 to 11 min; 95 % solvent B from 11 to 14 min (equilibration step). Mass spectrometry operated in positive ions selected reaction monitoring (SRM) mode, by using optimized source and compound parameters to achieve the best possible performances. Three transitions were used to monitor each compound and internal standard: that one with the highest signal/noise ratio was used as a quantifier (Q), while the other two as qualifiers (q). They were the following: MIT-But: 363.9 → 261.8 Da (Q), 363.9 → 135 Da (q), 363.9 → 291.0 Da (q); 13C9-MIT-But: 373.1 → 270.1 Da (Q), 373.1 → 143.2 Da (q), 373.1 → 300.2 Da (q); DIT-But: 489.9 → 387.9 Da (Q), 489.9 → 260.9 Da (q), 489.9 → 290.0 Da (q); 13C9-DIT-But: 498.8 → 395.8 Da (Q), 498.8 → 268.8 Da (q), 498.8 → 298.9 Da (q); T3-But: 707.9 → 605.9 Da (Q), 707.9 → 479.1 Da (q), 707.9 → 651.9 Da (q); 13C6-T3-But: 713.9 → 611.9 Da (Q), 713.9 → 485.1 Da (q), 713.9 → 657.9 Da (q); T4-But: 833.9 → 731.99 Da (Q), 833.9 → 605.0 Da (q), 833.9 → 777.9 Da (q); 13C6-T4-But: 839.9 → 737.9 Da (Q), 839.9 → 611.0 Da (q), 839.9 → 783.8 Da (q).

*Method validation:* the analytical method was validated according to standard criteria [20]. Linearity was evaluated within the calibration curve range. Sensitivity was assessed by evaluating limits of detection (LOD) and limits of quantification (LOQ) of the analytes, which corresponded to the concentrations providing an S/N ratio close to 3 and 10, respectively, measured with a specific tool of the instrument control and data processing software (ABSciex Analyst^{®} 1.7.2). Accuracy (%) was measured on plasma and urine samples spiked with 3 different concentration levels of analytes (C1, 2.5ng/mL, C2 10 ng/mL, C3 50 ng/mL), and the spiked concentration was determined by subtracting the endogenous concentration, C0, to C1, C2, C3. Accuracy (%) was calculated by the formula [(measured concentration/nominal concentration spiked) × 100]. Recovery was evaluated by comparing the peak areas of the internal standards added before and after the extraction procedure. It was expressed as % and calculated by the formula [(peak area of IS added before the extraction/peak area of IS added after the extraction) × 100]. The estimation of matrix effect (%) was evaluated by comparing the peak area of the internal standard added after the extraction in water (A) and matrix (B) and calculated by the formula [(B/A) × 100].

### UIC determination

UIC was determined by the Sandell-Kolthoff method modified by Benotti and Benotti (21). Calibration standards were prepared from a stock solution with an iodide concentration of 10,000 µg/L. The following controls were also prepared; low iodide (<30mg/L), high iodide (>300mg/L) and Biorad control. Ammonium persulfate was added followed by 1 h incubation at 100°C. Arsenious acid (reducing agent) and ammonium sulfate ceric (oxidant) were added producing a colour change. Using the spectrophotometer, absorbance values were obtained, and a ratio was calculated using urine creatinine levels of samples.

### 1.6. Statistical analysis

Statistical analysis for the experiments were performed using T-test in Prism software version 5.

### Example 2. Dehal1 inhibition capacity of xenobiotic compounds in vitro.

Twenty unlabeled phenolic-based compounds with/without halogen (Iodine, Bromine, Chlorine, Fluorine) substitutions were assayed at 10 µM concentration for their capacity to inhibit labelled-MIT deiodination by DEHAL1 enzyme including MIT and DIT natural substrates and mono-chloro-tyrosine (MCT) and MNT, together with empty pCDNA3 vector-transfected cell lysates, DMSO vehicle, NaOH and Buffer as negative controls (Figure 2A). TBBPA and PCP showed 37% and 55% inhibition of labeled-MIT deiodination, respectively. Moreover, MCT and MNT showed maximal inhibition of labeled-MIT deiodination at 10 µM, similar to natural substrates MIT and DIT. Furthermore, dose-response inhibition assays with increasing concentrations of compounds (0-100 µM) were performed for TPPBA, BPA (as non-halogenated control), PCP and MNT. TBBPA and PCP showed partial inhibition of Dehal1 activity (18% and 22 %) at 10 µM and 78% at 100 µM concentrations, showing IC₅₀ estimated at 25µM. BPA showed no inhibition capacity, suggesting that halogen phenolic substitution is a requirement for inhibition. MNT, as expected, showed IC₅₀ at concentrations lower than 0.01 µM. Thus, these results show for the first time that two halogenated phenolic compounds, TBBPA and PCP are partial inhibitors of Dehal1 *in vitro,* while MNT is a strong inhibitor of its enzymatic activity.

### Example 3. Molecular docking of inhibitors on the X-ray based crystallographic structure of Dehal1.

MIT was docked on the dimeric crystal structure of Dehal1 as control, showing its positioning at the two hydrophobic substrate-binding pocket available at the dimer surface, and interaction with Flavin-mononucleotide (FMN) cofactor (A). In detail, MIT establishes interactions with alanine 130 (A130), tyrosine 161 (Y161), lysine 182 (K182) and glutamic acid 157 (E157) while FMN interacts with threonine 239 (T239) and the amino group of MIT (B). TBBPA occupy the substrate binding pocket of the enzyme but losses the typical interactions with Dehal1 amino acids and the cofactor. MNT, however, is capable to interact with protein and cofactor similarly to the natural substrate MNT. These experiments shape TTBPA as typical partial inhibitors of Dehal1 at the molecular level, competing for the binding to the substrate pocket with MIT. MNT would behave as al alterative substrate to MIT, but also a a strong inhibitor of MIT deiodination, even more when MNT was shown to be present in environmental atmospheres, and could be considered an air pollutant (15).

Thus, the three compounds were shown for the first time to interact with the X-ray based crystallographic structure of the enzyme precisely in the substrate binding pocket of the dimer, profiling as typical competitive inhibitors of the Dehal1.

### Example 4. Hormonal and biochemical effects of in vivo administration of TBBPA and MNT to mice.

After confirmation of the physical interaction of such molecules with DEHAL1 dimer using molecular docking studies and the crystallographic model of the enzyme, the inventors proceeded towards the *in vivo* validation of such inhibition potential in a living rodent mode. In particular, TBBPA and MNT were administered at moderate and mild dose, respectively, to animal models *in vivo,* to study the possible disrupting effects of the chemicals on thyroid hormone axis.

### 4.1. TBBPA

Plasma TSH increased significantly after administration of 500 mg/kg b.w/day TBBPA while T4 decreased and T3 remained unchanged (Figure 4.A), indicating that the xenobiotic caused hypothyroidism in a relatively short time span. TBBPA-treated mice exhibited an increase in plasmatic MIT (25%), but a marked decrease in palsma DIT (Fig. 4A). In urine iodotyrosines follow the same divergent vaiation (MIT significantly increases and DIT decreases) (figure 4.B). Overall, the incerase in urine MIT leads to increases in the UIC. Therefore, MIT and DIT could be useful biomarkers of hypothyroidism caused by environmental TDCs, especially an increased MIT/DIT ratio should be very sensitive markers in detection that etiology of hypothyroidism.

**Table 1.**

| | **WT** | **WT-TBBPA** | **% variation** | **Statistical significance** |
|---|---|---|---|---|
| **TSH (mU/L)** | 1 | 1.3 ± 0.025 | **+23** | * |
| **T4 (ng/ml)** | 23.48 ± 2.39 | 14.45 ±1.65 | **-38** | * |
| **MIT-plasma (ng/ml)** | 0.16 ±0.01 | 0.21 ± 0.01 | **+23** | * |
| **DIT-plasma (ng/ml)** | 0.16 ± 0.01 | 0.06 ± 0.01 | **-62** | *** |
| **MIT-urine** (**ng/ml)** | 1.46 ± 0.16 | 2.53 ± 0.11 | **+73** | ** |
| **DIT-urine (ng/ml)** | 0.36 ± 0.08 | 0.23 ± 0.04 | **-36** | ns (p=0.23) |
| **UIC (ug/l)** | 74.7 ± 5.2 | 170.6 ± 13.9 | **+127** | * |
| **MIT/DIT plasma** | 1 | 3,5 | **+250** | *** |
| **MIT/DIT urine** | 4.05 | 11 | **+172** | ** |

| | | | | |
|---|---|---|---|---|
| For statistical significance: * p<0.05; **p<0.01; ***p<0.005. n.s.: no significant (p<0.05) | | | | |

TBBPA caused hypothyroidism (TSH elevation and T4 decrease) in a short time lapse, and increase of MIT in plasma and urine, as well as DIT decrease in both type of fluids was detected. During the experiment, mice were fed normal iodine containing diet, therefore, variations of iodotyrosines are attributed to inhibition of Dehal1 with lack of deiodination and secretion to the blood stream and the urine, causing iodine depletion. Probably, DIT synthesis (containing two iodine atoms) is down-regulated in the thyroid gland as a way to save iodine and preserve T3 synthesis and secretion (containing MIT+DIT) at the cost of reducing T4 synthesis and secretion (T4 is formed by coupling of two DIT molecules: DIT+DIT).

### 4.2. MNT

Given the strong inhibition of MNT on Dehal1 activity *in vitro,* we decided to administer a low concentration of MNT (1mg/Kg b.w./day, for 7 days) in mice. As shown in Table 2, at this low dose, TSH, T4 and T3 were still unchanged. Interestingly, MIT was significantly increased in urine of exposed mice, leading to also increased UIC, while DIT showed a non-significant tendency towards decrease in concentration. The results show that even very mild exposure to MNT, modifies MIT (and possibly DIT) concentrations in urine ( a more sensitive type of sample to determine low concentration of iodotyrosines, due to an accumulative effect of iodotyrosine secretion) before hypothyroidism ensues, suggesting that iodotyrosines could also be useful as biomarkers of mild TDCs exposure before the damage causes hypothyroidism, suggesting early biomarking value, and not also diagnostic value of the MIT/DIT determination in suspicion of environmental TDC exposures.

**Table 2.**

| | **WT** | **WT-MNT** | **% variation** | **Statistical significance** |
|---|---|---|---|---|
| **TSH (mU/L)** | **1** | **1.07 ± 0.07** | **+7** | **n.s** |
| **T4 (ng/ml)** | **23.48 ± 2.39** | **25.35 ± 1.2** | **+8** | **n.s** |
| **T3 (ng/ml)** | **0.285 ± 0.02** | **0.27 ± 0.02** | **-5** | **n.s** |
| **MIT-urine (ng/ml)** | 1.33 ± 0.17 | 1.94 ± 0.13 | **+46** | ***** |
| **DIT-urine (ng/ml)** | 0.36 ± 0.09 | 0.29 ± 0.06 | **-19** | **n.s** (p=0.5) |
| **UIC (ug/l)** | 135.4 ± 2.43 | 198 ± 14.43 | **+47** | ***** |
| **MIT/DIT urine** | 3,69 | 6,69 | **+80** | ****** |

| | | | | |
|---|---|---|---|---|
| For statistical significance: * p<0.05; **p<0.01; ***p<0.005. n.s.: no significant (p<0.05). | | | | |

## Claims

1. A method for detecting the exposure of a subject to a Thyroid Disrupting Chemical (TDC), comprising determining the concentration of mono-iodotyrosines (MIT) and di-iodotyrosines (DIT) in a biological sample taken from said subject, wherein an increase in the concentration of MIT and a decrease in the concentration of DIT in comparison to a reference is indicative of exposure to a TDC, or wherein an increased ratio MIT/DIT in comparison to a reference is indicative of exposure to a TDC.

2. A method for determining the cause of a non-genetic hypothyroidism comprising determining the concentration of MIT and DIT in a biological sample taken from a subject, wherein an increase in the concentration of MIT and a decrease in the concentration of DIT in comparison to a reference is indicative of said hypothyroidism being caused by exposure to a TDC, or wherein an increased ratio MIT/DIT in comparison to a reference is indicative of said hypothyroidism being caused by exposure to a TDC.

3. The method according to any one of the preceding claims, wherein the TDC is selected from the group consisting of tetrabromobisphenol A (TBBPA); 3-L-nitrotyrosine (MNT); pentachloro-phenol (PCP); 4-OH-2',3,4',5,6'-pentachlorobiphenyl; 4-OH-2',3,4',6-tetrachlorobiphenyl; 4-OH-2,2',5,5'-tetrachlorobiphenyl; 4,4'-diOH-3,3',5,5'-tetrachlorobiphenyl; 3-OH-2,2',5,5'-tetrachlorobiphenyl; 4'-OH-brominated diphenyl ether (BDE)-17; 4-OH-BDE-42; 4'-OH-BDE-49; 4-OH-BDE-90; 2-OH-BDE-15; 2'-OH-BDE-28; Bromoxynil; Oxyclozanide; Bithionol; Tribromsalan; Nitroxynil; Closantel; Benzbromarone; Triclosan; Rose Bengal; Erythrosine B; Phloxine B, Methylene blue; Tannic acid; Diquat dibromide monohydrate; D&C Red 27; Dodecylphenol; Lauryl gallate; 4-Dodecylphenol; 4-Nonylphenol; Dinocap; Hexadecyltrimethyl-ammonium bromide Sodium myristyl sulfate; Calcium dodecylbenzene sulfonate; 2,2'-Methylenebis(4-methyl-6-tert- butylphenol); Sodium hexyldecyl sulfate; Sodium tridecyl sulfate; Sodium dodecylbenzenesulfonate; Methylbenzethonium chloride; Docusate sodium; Kepone; Oleic acid; Dodecylbenzenesulfonic acid; Octylparaben; C.I. Acid Red 114; and combinations thereof.

4. The method according to any one of the preceding claims, wherein the TDC is TBBPA, MNT, PCP or combinations thereof.

5. The method according to any one of the preceding claims, wherein the TDC is TBBPA and/or MNT.

6. The method according to any one of the preceding claims, wherein the biological sample is urine and/or plasma, preferably urine.

7. The method according to any one of the preceding claims, further comprising determining the median urinary iodine concentration (UIC).

8. The method according to any one of the preceding claims, further comprising determining the concentration of thyroxine (T4) and triiodothyronine (T3).

9. The method according to the preceding claim, wherein the concentration of MIT and DIT, and optionally T4 and T3, is determined simultaneously by high performance liquid chromatography coupled to tandem mass spectroscopy (HPLC/MS-MS).

10. The method according to any one of the preceding claims, further comprising the determination of thyroid-stimulating hormone (TSH).

11. Use of a kit for carrying out the method of any one of claims 1 to 10, wherein the kit comprises:
a) appropriate means for determining the concentration of MIT, and
b) appropriate means for determining the concentration of DIT.

12. Use according to claim 11, wherein the kit further comprises appropriate means for determining the concentration of T4 and T3.

13. Use according to claim 11 or 12, wherein the kit further comprises appropriate means for determining UIC and/or TSH.

14. Use of a method according to any one of claims 1-10, or of a kit as defined in any one of claims 11-13 for *in vitro* TDC-caused hypothyroidism diagnosis.

15. Use of a method according to any one of claims 1-10, or of a kit as defined in any one of claims 11-13 for determination of the cause of non-genetic hypothyroidism or for detecting the exposure of a subject to a TDC.
